# EUROPEAN PATENT SPECIFICATION

(11) **EP 3 836 979 B1**
(45) Date of publication and mention of the grant of the patent: **25.10.2023**
(21) Application number: 19808635.7
(22) Date of filing: 16.08.2019
(51) Int. Cl.: A61L 27/20, A61L 27/26, A61L 27/36, A61L 27/38, A61L 27/56

(54) **METHOD OF PRODUCING THREE DIMENSIONAL AUTOLOGOUS FAT GRAFT USING HUMAN LIPOASPIRATE-DERIVED ADIPOSE TISSUE WITH MULTIPOTENT STEM CELLS AND BIOCOMPATIBLE CELLULOSE NANOFIBRILS**
VERFAHREN ZUR HERSTELLUNG VON DREIDIMENSIONALEN AUTOLOGEN FETTTRANSPLANTATEN UNTER VERWENDUNG VON MENSCHLICHEM FETTGEWEBE AUS LIPOASPIRAT MIT MULTIPOTENTEN STAMMZELLEN UND BIOKOMPATIBLEN CELLULOSE-NANOFIBRILLEN
PROCÉDÉ DE PRODUCTION D'UN GREFFON DE GRAISSE AUTOLOGUE 3D À L'AIDE DE TISSU ADIPEUX DÉRIVÉ DE LIPOASPIRAT HUMAIN AVEC DES CELLULES SOUCHES MULTIPOTENTES ET DES NANOFIBRILLES DE CELLULOSE BIOCOMPATIBLES

(30) Priority: 17.08.2018 US 201862719162 P
(43) Date of publication of application: 23.06.2021
(73) Proprietor: Ocean Tunicell AS, 5258 Blomsterdalen (NO)
(72) Inventor: GATENHOLM, Paul, Riner, VA 24149 (US)
(74) Representative: Forresters IP LLP
(86) International application number: PCT/IB2019/000922
(87) International publication number: WO 2020/035734

(56) References cited:
- WO-A1-2016/100856
- WO-A1-2018/055452
- US-A1- 2018 055 971
- KAJSA MARKSTEDT ET AL: "3D Bioprinting Human Chondrocytes with Nanocellulose-Alginate Bioink for Cartilage Tissue Engineering Applications", BIOMACROMOLECULES, vol. 16, no. 5, 11 May 2015 (2015-05-11), pages 1489-1496, XP055254675, US ISSN: 1525-7797, DOI: 10.1021/acs.biomac.5b00188

## Description

### CROSS-REFERENCE TO RELATED APPLICATIONS

The present application relies on the disclosures of and claims priority to and the benefit of the filing date of U.S. Provisional Application No. 62/719,162, filed August 17, 2018.

### BACKGROUND OF THE INVENTION

### Field of the Invention:

The present invention generally relates to autologous fat grafting and more particularly relates to a method of producing three dimensional autologous fat graft using human lipoaspirate-derived adipose tissue, including multipotent stem cells and biocompatible cellulose nanofibrils derived from tunicates.

### Description of Related Art:

Autologous fat grafting is an established reconstructive and aesthetic surgical procedure because of the abundance of fat tissue and the relative simplicity of the procedure. The applications of fat grafting include facial rejuvenation, hand rejuvenation, breast reconstruction and volume enhancement, treatment of skin photoaging, correction of contour deformities, and improvement of senile and diabetic plantar fat pad atrophy. The process typically consists of three steps: harvesting of adipose tissue from a suitable donor site; processing of the lipoaspirate; and reinjection of the purified adipose tissue. The outcome is, however, poor and unpredictable. The main problem after autologous adipose tissue transplantation is its absorption rate over time, which results in an average reduction from 25% to 70% of the total implanted volume (see, Simonacci, F., Bertozzi, N., Grieco, M. P., Grignaffini, E. and Raposio, E., Procedure, applications, and outcomes of autologous fat grafting. Annals of Medicine and Surgery 20, 49-60 (2017)). Adipose cells die and become resorbed and the empty space allow infiltration of macrophages and polynucleated cells for phagocytosis. Grafted adipocytes pass through an ischemic phase. Vascularization has been observed when the fat fragments are very small.

Further, fat tissue is highly vascularized and contains several types of cells in addition to adipocytes. There is a population of pericytes close to a vascular network, endothelial progenitor cells, and adipose stem cells which are mesenchymal stem cells. The isolation of Stromal Vascular Fraction (SVF) from lipoaspirate is possible through digestion of an extracellular matrix along with several enzymes of which collagenase is typically the most important. SVF can be isolated by a manual procedure or by an automated procedure using commercially available automation equipment which enzymatically digest extracellular matrix, and wash and separate (examples include: Cytori Celution 800/CRS System, Medi-Khan's Lipokit and Icellator from Tissue Genesis). Adipose derived Stem cells (ASC) can then be plated out from SVF fraction and further expanded. SVF has been recognized as a potent cell fraction with the capability of healing and promoting vascularization. Isolation of SVF is also, however, associated with use of digestive enzymes which might have adverse effects for patients, and, at the time of this application, the regulatory approval is still pending. Other procedures of fat disintegration without use of enzymes include mechanical treatment, which is applied for example in a device from Lipogems, Italy (see Bianchi, F. et al., A new nonenzymatic method and device to obtain a fat tissue derivative highly enriched in pericyte-like elements by mild mechanical forces from human lipoaspirates. Cell Transplant. 22, 2063-2077 (2013); Tremolada, C., Colombo, V. & Ventura, C., Adipose tissue and mesenchymal stem cells: state of the art and Lipogems® technology development. Current stem cell reports 2, 304-312 (2016)).

A challenge met by the current invention is to improve survival of a fat graft, which, if can be achieved, results in the autologous fat grafting, which can be effectively used for wound healing and reconstructive and aesthetic surgery.

According to the invention herein, a possible way to improve survival of a fat graft would be to encapsulate it in a biopolymer matrix in such a pattern to create porosity providing for the ability of diffusion of nutrients and oxygen, as well as facilitating vascularization. Several biopolymers have been used as scaffolds and bioinks for general tissue engineering (see, e.g., Domingues, R. M. A., et al., Development of Injectable Hyaluronic Acid/Cellulose Nanocrystals Bionanocomposite Hydrogels for Tissue Engineering Applications, Bioconjugate Chem., 2015, 26 (8), pp. 1571-1581; Gatenholm, P., Cellulose nanofibrillar bioink for 3d bioprinting for cell culturing, tissue engineering and regenerative medicine applications, Patent Application, WO2016100856A1) wherein the biomaterials are combined with single cells. Polymer solutions are shear thinning, which means that the viscosity is decreased with increased shear rate. Cellulose powder composed of cellulose nanocrystals has been proposed for use in tissue engineering as described in patent application WO2007066222 A1, but such material does not provide sufficient nanofibrillar architecture.

Cellulose nanofibrils (CNF), which can be isolated from tunicates, produced by bacteria or isolated from primary or secondary cell walls of plants, are 8-30 nm in diameter and can be up to a micrometer long. They have a hydrophilic surface and therefore bind water on their surfaces forming hydrogels already at low solid content (1-2%). CNF are shear thinning and have high zero shear viscosity. The hydrophilic nature of the CNF surfaces covered by water prevent them from protein adsorption and make them bioinert. Cells do not recognize CNF surfaces which are an advantage, as taught herein, when it comes to biocompatibility since there is little to no foreign body reaction. Among various cellulose nanofibrils, tunicate derived nanofibrils have high crystallinity and thick fibril dimension with fibril lengths that can exceed 1 micrometer. According to the invention herein, this plays a role in dispersing power and viscoelastic properties. Nanocellulose fibrils combined with crosslinked alginate or hyaluronate have been shown to act as a suitable scaffold for differentiation of adipose progenitor cells (see, e.g., Henriksson, I., P. Gatenholm, and D.A. Hagg, Increased lipid accumulation and adipogenic gene expression of adipocytes in 3D bioprinted nanocellulose scaffolds. Biofabrication, 2017, Feb 21;9(1); Krontiras, P., P. Gatenholm, and D.A. Hagg, Adipogenic differentiation of stem cells in three-dimensional porous bacterial nanocellulose scaffolds. Journal of Biomedical Materials Research Part B-Applied Biomaterials, 2015. 103(1): p. 195-203) but not in combination with adipose cells. Patent application US2018/0055971 A1 discloses a soft tissue augmentation product comprising a lipoaspirate mixed with hyaluronic acid and silk fibroin hydrogels.

### SUMMARY OF THE INVENTION

The invention is defined by the claims. Any subject-matter falling outside the scope of the claims is provided for information purposes only. Any reference in the description to methods of treatment refer to the products of the present invention for use in a method for treatment of the human (or animal) body by therapy.

The invention herein overcomes the above challenges by introducing a method of producing a three dimensional autologous fat graft using human lipoaspirate-derived adipose tissue with multipotent stem cells and biocompatible cellulose nanofibrils derived from tunicates. In a preferred embodiment, the three dimensional autologous fat graft using human lipoaspirate-derived adipose tissue is rich in or comprises a high amount or content of multipotent stem cells and biocompatible cellulose nanofibrils derived from tunicates.

One embodiment herein provides a method of producing three dimensional autologous fat graft(s) with porosity, which survives implantation without volumetric shrinkage and becomes neovascularized to be used in aesthetic and reconstructive surgery in which the lipoaspirate, preferably rich in stem cells, is mixed with one or more excipients and/or biocompatible material(s) comprising nanofibrillar cellulose derived from tunicates, and crosslinked.

Another embodiment provides a method of producing three dimensional autologous fat graft(s) with porosity, which survives implantation without volumetric shrinkage and becomes neovascularized to be used in aesthetic and reconstructive surgery in which the lipoaspirate, preferably rich in stem cells, is mixed with one or more biocompatible material(s) comprising nanofibrillar cellulose derived from tunicates, and crosslinked.

In another embodiment, the invention herein comprises a method of producing a three dimensional autologous fat graft with porosity, which survives implantation without volumetric shrinkage and becomes neovascularized, to be used in aesthetic and reconstructive surgery, in which lipoaspirate, preferably rich in stem cells, is mixed with one or more excipient and/or one or more biocompatible material(s) comprising nanofibrillar cellulose derived from tunicates, which is crosslinked. In aspects, the nanofibrillar cellulose derived from tunicates is in the form of dispersion with fibrils of a diameter above 15 nm and length between 0.5 microns and 5 microns. In another embodiment, the nanofibrillar cellulose is derived from tunicates in the form of dispersion with solid content higher than 1.5% and lower than 4% by weight. The nanofibrillar cellulose derived from tunicates, in aspects, has no detectable hemicelluloses that affects immunoresponse and little to no detectable agglomerates. In an embodiment, nanofibrillar cellulose derived from tunicates is in the form of dispersion with no little to no detectable bacteria derived lipopolysaccharides known in cases as endotoxins.

In another embodiment, the nanofibrillar cellulose derived from tunicates is crosslinked by addition of alginate and Calcium, Barium and/or Strontium ions. In another embodiment, nanofibrillar cellulose derived from tunicates is crosslinked by addition of alginate and polylysine. In another embodiment, the nanofibrillar cellulose derived from tunicates is crosslinked by addition of a solution fibrinogen and/or thrombin.

In an embodiment, the nanofibrillar cellulose derived from tunicates is crosslinked by addition of platelet rich plasma (PRP) followed by or preceded by addition of Calcium ions. In another embodiment, the nanofibrillar cellulose derived from tunicates is crosslinked by addition of riboflavin 5'-phosphate followed by or preceded by exposure to UV (ultraviolet) light.

Another embodiment comprises an implantable tissue prepared for use in aesthetic and reconstructive surgery of a human. In an embodiment, an implantable tissue is prepared for use in aesthetic and reconstructive surgery of animals, including humans or other animals. In another embodiment, an implantable tissue is prepared for wound healing.

### BRIEF DESCRIPTION OF THE DRAWINGS

The accompanying drawings illustrate certain aspects of embodiments of the present invention and should not be used to limit the invention. Together with the written description the drawings explain certain principles of the invention.
Figures 1A-C show various steps of lipoaspirate collection and processing techniques.
Figure 2 shows rheological properties of disintegrated lipoaspirate (L), mixed with alginate (LA), mixed with alginate and nanocellulose from tunicates (LT).
Figure 3 shows crosslinking kinetics measurements.
Figures 4A-B show results from 3D shaping experiments.
Figures 5A-D show results from in vivo experiments.

### DETAILED DESCRIPTION OF VARIOUS EMBODIMENTS

Reference will now be made in detail to various exemplary embodiments of the invention. It is to be understood that the following discussion of exemplary embodiments is not intended as a limitation on the invention. Rather, the following discussion is provided to give the reader a more detailed understanding of certain aspects and features of the invention.

The embodiments herein and the various features and advantageous details thereof are explained more fully with reference to the non-limiting embodiments that are illustrated in the accompanying drawings and detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

The embodiments herein comprise a method based on mixing mechanically disintegrated lipoaspirate, enriched with stem cells, with biopolymers to achieve partially or fully homogenous dispersion, and then processing the mixture/using the mixture to create three dimensional architecture with porosity. The biopolymer materials, in aspects, are used as a dispersant/emulsifier. Porosity according to the current invention enables diffusion of nutrients and/or oxygen and contributes to survival of adipose cells, but it also allows for differentiation of progenitor cells and neovascularization. Grafting according to the present invention can be implemented by techniques, including but not limited to moulding, 3D Bioprinting, or injection into a human or animal body. Biopolymers described herein have, in aspects two different functions; first, providing homogeneous dispersion with shear thinning properties (pseudoplastic), and second crosslinking. Homogeneous dispersion/emulsion is achieved by adding biocompatible cellulose nanofibrils derived from tunicates and other excipient(s), and crosslinking is achieved for example by using fibrinogen and thrombin or alginate and calcium, addition of platelet rich plasma (PRP) followed by addition of Calcium ions, and/or addition of riboflavin 5'-phosphate followed by exposure to UV light. Cellulose nanofibrils isolated from tunicates, are used as dispersing and emulsifying agent(s), but they may also provide shear thinning properties, high viscosity at low shear stresses, and mechanical stability of the 3D fat graft. They also may enable improved porosity, which may result in neovascularization or improved neovascularization. Such described 3D autologous fat graft, in a preferred embodiment, survives implantation and does not volumetrically shrink to a medical meaningfully degree. It also gets vascularized in a preferred embodiment. The invention in this manner provides an innovative way to heal wounds and enable corrections and repair of soft tissue in reconstructive and aesthetic surgery.

### Example 1 (Lipoaspirate collection and processing)

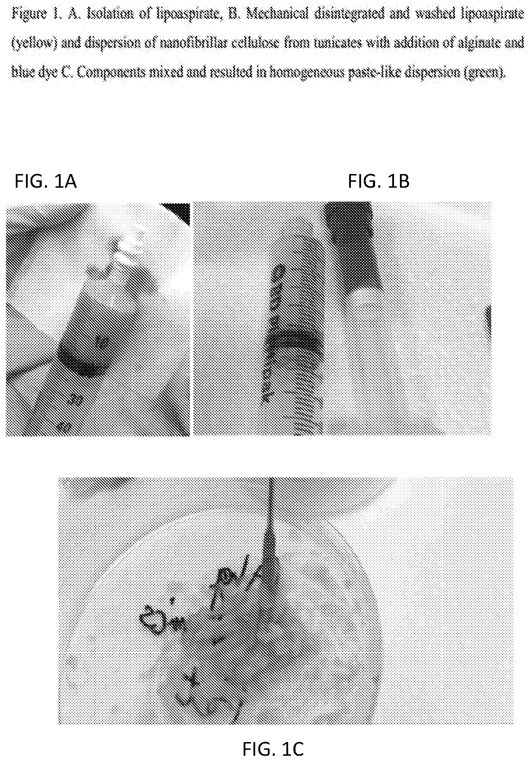

Liposuction aspirate was collected from the abdominal subcutaneous tissue of a healthy female donor with approval from the Regional Ethics Committee of Gothenburg (Dnr 624-16) and after signing informed consent. Lipoaspirate was processed with a Lipogems device, Italy, according to the manufacturer's protocol by mechanical force and without any enzymatic treatment. Briefly, lipoaspirate was washed with a physiologic electrolyte solution (Ringer's Acetate, Braun), emulsified by shaking with metal beads 5x20s, and rinsed in a closed system. A total of 160 ml of lipoaspirate was used from the donor. The processed lipoaspirate-derived adipose tissue described previously was mixed with 3% by weight solution of alginate SLG100 (Novamatrix, Norway) in 4.5% mannitol solution at a volumetric ratio of 90:10. The adipose mixture was further mixed with nanocellulose/alginate (80:20) in a 1:1 ratio. Figure 1A shows the isolated lipoaspirate and Figure 1B shows the processed form with Lipogems device lipoaspirate (yellow) and dispersion of cellulose nanofibrils from tunicates with added alginate and blue dye (blue). Figure 1C shows green homogenous dispersion with paste-like consistency when processed lipoaspirate was mixed with cellulose nanofibril dispersion.

### Example 2 (Rheology)

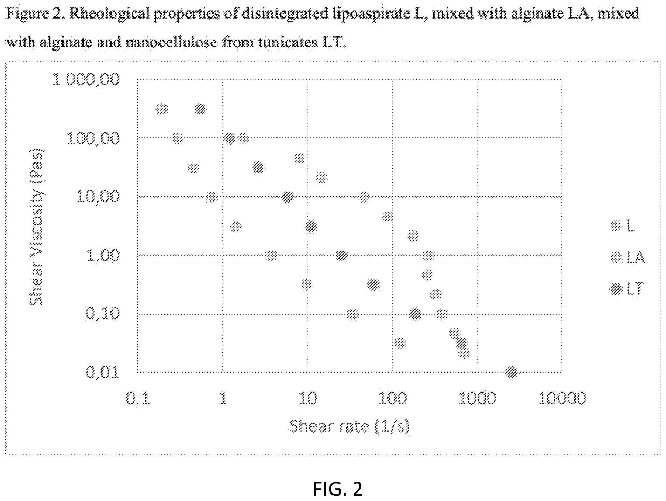

The rheological properties and crosslinking kinetics of the lipoaspirate-derived adipose disintegrated tissue prepared by the Lipogems system (L), and the processed adipose tissue mixed with alginate (LA) and alginate and nanocellulose from tunicates (LT), were analysed using a Discovery HR-2 rheometer (TA Instruments, UK) equipped with a peltier plate. All measurements were conducted at 25°C. Shear viscosity was measured at shear rates from 0.01 - 1500 s-1 with a plate-plate geometry (20 mm, gap = 500 µm). The mechanically processed lipoaspirate-derived adipose tissue (L) was inhomogeneous and consisted of clusters of adipose tissue and free flowing liquid. The viscosity curve for L was non-linear due to the inhomogeneity of the material (see Figure 2, upper curve). Addition of alginate provided more homogeneous dispersion with shear thinning properties (see lowest curve in Figure 2). Viscosity was, however, too low for optimal 3D shaping. Addition of tunicate derived cellulose nanofibrils resulted in shear thinning properties and viscosity allowing 3D shaping (see curve LT in Figure 2).

### Example 3 (Crosslinking)

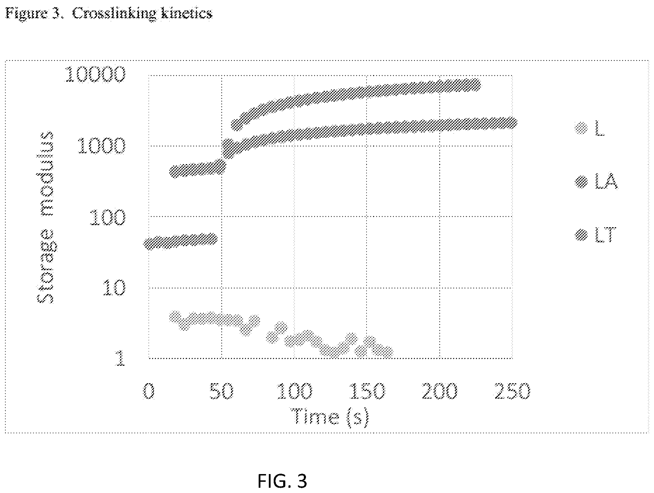

The crosslinking experiments were performed to evaluate different crosslinkers and also kinetics of crosslinking. Figure 3 shows results from experiments when alginate was used and crosslinking was performed with addition of 0.1 M CaCh solution. Oscillation frequency measurements were conducted at stresses within the measured LVR region and at a frequency range of 0.1 - 100 rad/s. Oscillation time measurements were conducted at 1.5% strain and a frequency of 1Hz for 10 minutes. 20 seconds after initiating the measurement, 1 ml of 0.1 M CaCl2 was dispensed around the measured lipoaspirate dispersions while collecting data on the storage modulus. The lowest curve in Figure 3 was lipoaspirate (L) and it was not crosslinked by addition of CaCh solution. In contrast, both lipoaspirate with alginate (LA) and with alginate and cellulose nanofibrils isolated from tunicates were crosslinked within 60-100 seconds, as seen by the quick increase in moduli upon addition of CaCh at t=40s. It can be seen in Figure 3 that the storage modulus of crosslinked lipoaspirate is higher when cellulose nanofibrils from tunicates are added compared only with alginates. Additional crosslinking experiments were performed, wherein instead of an alginate and CaCh solution, a fibrinogen and thrombin solution was added. The dispersion crosslinked fast after the thrombin addition. Addition of platelet rich plasma (PRP) followed by addition of Calcium ions also provided crosslinking. Addition of riboflavin 5'-phosphate followed by exposure to UV was also performed and resulted in crosslinking.

### Example 4 (3D shaping: Moulding and 3D bioprinting)

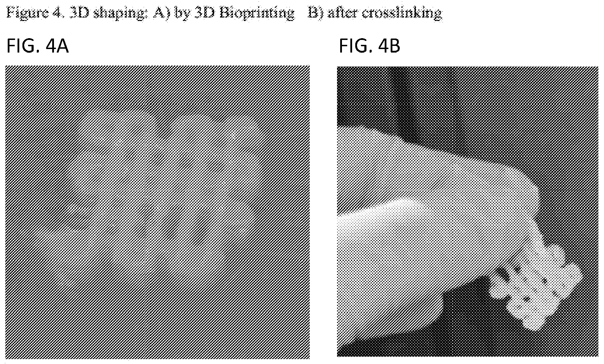

3D shaping ability of the lipoaspirate-derived adipose tissue processed by the Lipogems system solely (L), or mixed with alginate (LA), and alginate and nanocellulose (LT), respectively, was evaluated by moulding and printing with the pneumatic extrusion based 3D bioprinter "Inkredible" (Cellink AB, Sweden). Grid designs were printed with a conical nozzle (diameter 800 micrometer). The ability to dispense the material continuously through the nozzle and obtain printed structures with a printing fidelity for which the grid design was visible was evaluated. Neither lipoaspirate alone (L) nor with alginate (LA) was optimally able to be shaped into 3D structures. Only lipoaspirate with addition of cellulose nanofibrils derived from tunicates was optimally processed/formed into 3D structures, as can be seen in Figure 4. Figure 4A shows the result of grid architecture before crosslinking, and Figure 4B shows results after crosslinking. The 3D structure can be lifted and implanted in a preferred embodiment.

### Example 5 (Implantation of 3D grafts)

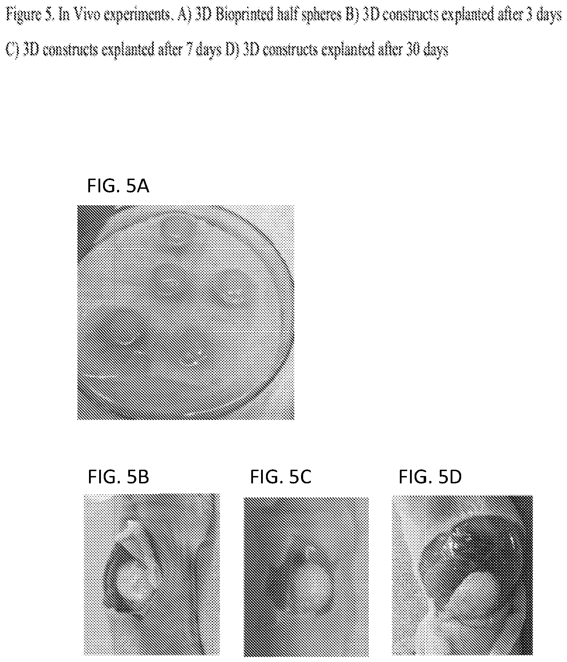

For in vivo experiments, the half spheres (diameter of 10 mm, height of 3 mm) were 3D Bioprinted, crosslinked with CaCh for 5 minutes and thereafter immediately implanted subcutaneously in BALB/c nude mice. Figure 5A shows 3D Bioprinted half spheres as they were 3D Bioprinted and crosslinked. Figure 5B shows 3D constructs in mice after 3 days; the 3D shape of the implanted half sphere has mostly not changed. Figure 5C shows 3D constructs after 7 days of implantation; there is mostly no change in volume of the construct and it appears as if it has become more elastic. Some vascularization was observed on the surface of explant. Figure 5D shows 3D constructs after 30 days implantation; there is little to no volume contraction. Opposite, the construct seems to increase slightly in volume, become elastic, change colour into more yellow, and partially or fully vascularize.

When an embodiment refers to "comprising" certain features, it is to be understood that the embodiments can alternatively "consist of' or "consist essentially of" any one or more of the features.

It is noted in particular that where a range of values is provided in this specification, each value between the upper and lower limits of that range, to the tenth of the unit disclosed, is also specifically disclosed. Any smaller range within the ranges disclosed or that can be derived from other endpoints disclosed are also specifically disclosed themselves. The upper and lower limits of disclosed ranges may independently be included or excluded in the range as well. The singular forms "a," "an," and "the" include plural referents unless the context clearly dictates otherwise.

## Claims

1. A method of producing a three dimensional autologous fat graft using human or animal lipoaspirate-derived adipose tissue, wherein the lipoaspirate comprises stem cells, wherein the lipoaspirate is mixed with nanofibrillar cellulose derived from tunicates, wherein the mixture of the lipoaspirate and the nanofibrillar cellulose derived from tunicates is crosslinked.

2. The method according to claim 1, wherein the nanofibrillar cellulose derived from tunicates, comprises a dispersion comprising fibrils with a length between 0.1 microns and 5 microns; preferably, a dispersion comprising fibrils having a length between 0.5 microns and 5 microns.

3. The method according to claim 1, wherein the nanofibrillar cellulose derived from tunicates comprises a dispersion having a solid content greater than 1.5% and less than 4% by weight.

4. The method according to claim 1, wherein the nanofibrillar cellulose derived from tunicates comprises a dispersion having an amount of hemicellulose that is lower than an amount that will substantially affect a human's immunoresponse system.

5. The method according to claim 1, wherein the nanofibrillar cellulose derived from tunicates comprises a dispersion having no detectable agglomerates.

6. The method according to claim 1, wherein the nanofibrillar cellulose derived from tunicates comprises a dispersion having no detectable bacteria derived lipopolysaccharides.

7. The method according to claim 1, wherein the nanofibrillar cellulose derived from tunicates is crosslinked by adding (a) alginate and (b) Calcium, Barium, and/or Strontium ions.

8. The method according to claim 1, wherein the nanofibrillar cellulose derived from tunicates is crosslinked by adding alginate and polylysine or other polycations.

9. The method according to claim 1, wherein the nanofibrillar cellulose derived from tunicates is crosslinked by adding a solution of fibrinogen and thrombin.

10. The method according to claim 1, wherein the nanofibrillar cellulose derived from tunicates is carboxymethylated or oxidized and then crosslinked by adding Calcium, Barium, and/or Strontium ions or polylysine or other polycations.

11. The method according to claim 1, wherein the nanofibrillar cellulose derived from tunicates is crosslinked by adding platelet rich plasma (PRP), followed by adding Calcium ions.

12. The method according to claim 1, wherein the nanofibrillar cellulose derived from tunicates is crosslinked by adding riboflavin 5'-phosphate, followed exposing to ultraviolet light.

13. An implantable composition comprising a three dimensional autologous fat graft comprising human or animal lipoaspirate-derived adipose tissue, wherein the lipoaspirate is mixed with nanofibrillar cellulose derived from tunicates and where the lipoaspirate comprises stem cells for use in aesthetic and/or reconstructive surgery of a human.

14. An implantable composition comprising a three dimensional autologous fat graft comprising human or animal lipoaspirate-derived adipose tissue, wherein the lipoaspirate is mixed with nanofibrillar cellulose derived from tunicates and where the lipoaspirate comprises stem cells for use in wound healing.

15. The method according to claim 1, wherein the three dimensional autologous fat graft is porous and survives implantation without substantial volumetric shrinkage; and/or wherein the three dimensional autologous fat graft becomes vascularized and is used for aesthetic and/or reconstructive surgery.

## Patentansprüche

1. Verfahren zum Herstellen eines dreidimensionalen Eigenfetttransplantats unter Verwendung von menschlichem oder tierischem von Lipoaspirat stammendem adipösem Gewebe, wobei das Lipoaspirat Stammzellen umfasst, wobei das Lipoaspirat mit nanofibrillärer Cellulose, die von Manteltieren stammt, gemischt wird, wobei die Mischung aus dem Lipoaspirat und der nanofibrillären Cellulose, die von Manteltieren stammt, vernetzt ist.

2. Verfahren nach Anspruch 1, wobei die nanofibrilläre Cellulose, die von Manteltieren stammt, eine Dispersion, umfassend Fibrillen mit einer Länge zwischen 0,1 Mikrometer und 5 Mikrometer; vorzugsweise eine Dispersion, umfassend Fibrillen, die eine Länge zwischen 0,5 Mikrometer und 5 Mikrometer aufweisen, umfasst.

3. Verfahren nach Anspruch 1, wobei die nanofibrilläre Cellulose, die von Manteltieren stammt, eine Dispersion aufweist, die einen Feststoffgehalt von größer als 1,5 Gew.-% und kleiner als 4 Gew.-% aufweist.

4. Verfahren nach Anspruch 1, wobei die nanofibrilläre Cellulose, die von Manteltieren stammt, eine Dispersion umfasst, die eine Menge an Hemicellulose aufweist, die niedriger als eine Menge, die ein Immunreaktionssystem eines Menschen im Wesentlichen beeinflussen wird, ist.

5. Verfahren nach Anspruch 1, wobei die nanofibrilläre Cellulose, die von Manteltieren stammt, eine Dispersion, die keine nachweisbaren Agglomerate aufweist, umfasst.

6. Verfahren nach Anspruch 1, wobei die nanofibrilläre Cellulose, die von Manteltieren stammt, eine Dispersion, die keine nachweisbaren von Bakterien stammenden Lipopolysaccharide aufweist, umfasst.

7. Verfahren nach Anspruch 1, wobei die nanofibrilläre Cellulose, die von Manteltieren stammt, durch Zugeben von (a) Alginat und (b) Calcium, Barium und/oder Strontium ionen vernetzt wird.

8. Verfahren nach Anspruch 1, wobei die nanofibrilläre Cellulose, die von Manteltieren stammt, durch Zugeben von Alginat und Polylysin oder anderen Polykationen vernetzt wird.

9. Verfahren nach Anspruch 1, wobei die nanofibrilläre Cellulose, die von Manteltieren stammt, durch Zugeben einer Lösung von Fibrinogen und Thrombin vernetzt wird.

10. Verfahren nach Anspruch 1, wobei die nanofibrilläre Cellulose, die von Manteltieren stammt, carboxymethyliert oder oxidiert und dann durch Zugeben von Calcium, Barium und/oder Strontiumionen oder Polylysin oder anderen Polykationen vernetzt wird.

11. Verfahren nach Anspruch 1, wobei die nanofibrilläre Cellulose, die von Manteltieren stammt, durch Zugeben von plättchenreichem Plasma (PRP) vernetzt wird, gefolgt durch Zugeben von Calciumionen.

12. Verfahren nach Anspruch 1, wobei die nanofibrilläre Cellulose, die von Manteltieren stammt, durch Zugeben von Riboflavin 5'-Phosphat vernetzt wird, einem Aussetzen gegen ultraviolettes Licht gefolgt.

13. Implantierbare Zusammensetzung, umfassend ein dreidimensionales Eigenfetttransplantat, umfassend menschliches oder tierisches von Lipoaspirat stammendes Fettgewebe, wobei das Lipoaspirat mit nanofibrillärer Cellulose, die von Manteltieren stammt, gemischt wird und wobei das Lipoaspirat Stammzellen zur Verwendung in ästhetischer und/oder rekonstruktive Chirurgie eines Menschen umfasst.

14. Implantierbare Zusammensetzung, umfassend ein dreidimensionales Eigenfetttransplantat, umfassend menschliches oder tierisches von Lipoaspirat stammendes Fettgewebe, wobei das Lipoaspirat mit nanofibrillärer Cellulose, die von Manteltieren stammt, gemischt wird, und wobei das Lipoaspirat Stammzellen zur Verwendung bei einer Wundheilung umfasst.

15. Verfahren nach Anspruch 1, wobei das dreidimensionale Eigenfetttransplantat porös ist und eine Implantation ohne wesentliche volumetrische Schrumpfung überlebt; und/oder wobei das dreidimensionale Eigenfetttransplantat vaskularisiert wird und für ästhetische und/oder rekonstruktive Chirurgie verwendet wird.

## Revendications

1. Procédé de production d'une greffe de graisse autologue tridimensionnelle à l'aide d'un tissu adipeux dérivé de lipoaspirat humain ou animal, dans lequel le lipoaspirat comprend des cellules souches, le lipoaspirat étant mélangé avec de la cellulose nanofibrillaire dérivée de témoins, le mélange du lipoaspirat et de la cellulose nanofibrillaire dérivée de tuniciers étant réticulé.

2. Procédé selon la revendication 1, dans lequel la cellulose nanofibrillaire dérivée de tuniciers comprend une dispersion comprenant des fibrilles ayant une longueur comprise entre 0,1 micron et 5 microns ; de préférence, une dispersion comprenant des fibrilles ayant une longueur comprise entre 0,5 micron et 5 microns.

3. Procédé selon la revendication 1, dans lequel la cellulose nanofibrillaire dérivée de tuniciers comprend une dispersion ayant une teneur en solides supérieure à 1,5 % et inférieure à 4 % en poids.

4. Procédé selon la revendication 1, dans lequel la cellulose nanofibrillaire dérivée de tuniciers comprend une dispersion ayant une quantité d'hémicellulose qui est inférieure à une quantité qui affectera sensiblement le système immunoréactif humain.

5. Procédé selon la revendication 1, dans lequel la cellulose nanofibrillaire dérivée de tuniciers comprend une dispersion ne présentant pas d'agglomérats détectables.

6. Procédé selon la revendication 1, dans lequel la cellulose nanofibrillaire dérivée de tuniciers comprend une dispersion n'ayant pas de lipopolysaccharides dérivés de bactéries détectables.

7. Procédé selon la revendication 1, dans lequel la cellulose nanofibrillaire dérivée de tuniciers est réticulée en ajoutant des ions (a) alginate et (b) Calcium, baryum, et/ou Strontium.

8. Procédé selon la revendication 1, dans lequel la cellulose nanofibrillaire dérivée de tuniciers est réticulée par ajout d'alginate et de polylysine ou d'autres polycations.

9. Procédé selon la revendication 1, dans lequel la cellulose nanofibrillaire dérivée de tuniciers est réticulée par ajout d'une solution de fibrinogène et de thrombine.

10. Procédé selon la revendication 1, dans lequel la cellulose nanofibrillaire dérivée de tuniciers est carboxyméthylée ou oxydée puis réticulée par ajout d'ions de calcium, de baryum et/ou de strontium ou de polylysine ou autres polycations.

11. Procédé selon la revendication 1, dans lequel la cellulose nanofibrillaire dérivée de tuniciers est réticulée par ajout de plasma riche en plaquettes (PRP), suivie de l'ajout d'ions calcium.

12. Procédé selon la revendication 1, dans lequel la cellulose nanofibrillaire dérivée de tuniciers est réticulée par ajout de riboflavine 5'-phosphate, suivi d'une exposition à la lumière ultraviolette.

13. Composition implantable comprenant une greffe de graisse autologue tridimensionnelle comprenant un tissu adipeux de lipoaspirat humain ou animal, dans laquelle le lipoaspirat est mélangé avec de la cellulose nanofibrillaire dérivée de tuniciers et où le lipoaspirat comprend des cellules souches destinées à être utilisées dans une chirurgie esthétique et/ou réparatrice d'un humain.

14. Composition implantable comprenant une greffe de graisse autologue tridimensionnelle comprenant du tissu adipeux de lipoaspirat humain ou animal, dans laquelle le lipoaspirat est mélangé avec de la cellulose nanofibrillaire dérivée de tuniciers et où le lipoaspirat comprend des cellules souches destinées à être utilisées dans la cicatrisation de plaies.

15. Procédé selon la revendication 1, dans lequel la greffe de graisse autologue tridimensionnelle est poreuse et survit l'implantation sans rétrécissement volumétrique substantiel ; et/ou dans lequel la greffe de graisse autologue tridimensionnelle devient vascularisée et est utilisée pour une chirurgie esthétique et/ou réparatrice.
